# EUROPEAN PATENT APPLICATION

(11) **EP 2 518 193 A2**
(43) Date of publication of application: **31.10.2012**
(21) Application number: 12167651.4
(22) Date of filing: 15.11.2007
(51) Int. Cl.: C40B 50/14, G01N 33/543, G01N 33/82

(54) **Methods for reversibly binding a biotin compound to a support**

(30) Priority: 15.11.2006 US 866021 P
(62) Divisional of application: 07874286.3
(71) Applicant: Invitrogen Dynal AS, 0379 Oslo (NO)
(72) Inventor: Brekke, Ole, 0476 Oslo (NO); Norderberg, Lars, 1450 Nesoddtangen (NO); Neurauter, Axl, 0853 Oslo (NO); Songe, Pal, 1176 Oslo (NO)
(74) Representative: Weber, Birgit

(57) **Abstract**

Methods of reversal of the binding between a biotin compound and a biotin-binding compound are disclosed. A method of reversibly releasing a biotinylated moiety from a streptavidin (or avidin) coated support is shown as an example. The strong interaction between streptavidin or avidin-biotin is made much weaker by using a combination of modified streptavidin or avidin and modified biotin like desthiobiotin or a derivative thereof like DSB-X Biotin. A protein, such as an antibody may be biotinylated with the modified biotin. When this protein is isolated by binding the modified biotin to the modified streptavidin or avidin bound to an solid surface, it may be released under very gently and very rapid conditions by addition of free biotin. In contrast to proteins obtained by the prior art release methods the protein obtained using the previously available release methods, the proteins obtained using the methods disclosed herein will maintain their native conformation. Uses of the methods in various procedures including cell detachment procedures and techniques of detection, identification, determination, purification, separation and/or isolation of target proteins or nucleic acid molecules are also described.

## Description

### CROSS REFERENCE TO RELATED APPLICATIONS

The present application claims priority to U.S. Provisional Patent Application Serial No. 60/866021, filed November 15, 2006.

### FIELD OF THE INVENTION

The present invention provides a gentle method of reversal of the binding between a biotin compound and a biotin-binding compound. In particular, the invention relates to a method of reversibly releasing a modified biotinylated moiety from a nitro-streptavidin (or avidin) coated support.

### DESCRIPTION OF RELATED ART

There is a continuous need in medical practice, research and diagnostic procedures for rapid, accurate, isolation or quantitative determination of different types of cells from various biological fluids.

A commonly used method to achieve such isolation or determination is through the formation of a linkage between two entities, an isolation or detection entity and the target. Such linkage is often accomplished using affinity binding, that is, by means of a pair of binding partners. One member of the pair is attached to or is part of the detection or isolation entity and the second is attached to or is part of the target. Linking occurs when the two parts are brought into contact. A number of binding partner systems are known, for example antigen-antibody, enzyme-substrate, ligand-receptor interactions on cells and biotin-streptavidin or avidin binding. Although the formation of such linkages between entities allows for such isolation, purification or immobilization, problems often arise if it is desired to further manipulate the isolated or purified cell population by breaking or reversing the linkage.

The avidin-biotin system, and in particular the streptavidin-biotin system, is one of the most widely used affinity bindings in molecular, immunological and cellular assays (Wilchek, M. and Bayer, E.A., Methods Enzymol. 184: 5-13 and 14-45, 1990. In general, a target molecule to be purified or detected is bound either directly to biotin or to a biotinylated intermediate. Such intermediate may be almost any molecule or macromolecule that will complex with or conjugate to a target molecule. For example, if a particular antigen, or epitope is the target, its binder would be an antibody or paratope-containing fragment thereof. The biotinylated target is bound to streptavidin which may be bound to a solid phase for ease of detection.

Streptavidin can also readily be immobilized on surfaces to capture, separate or detect biotinylated moieties, e.g. biotinylated molecules or biotin-labeled cells from crude, complex mixtures. This basic streptavidin-biotin interaction technique is utilized in affinity chromatography, cytochemistry, histochemistry, pathological probing, immunoassays, in-situ hybridization, bio-affinity sensors and cross-linking agents, as well as in more specific techniques such as targeting, drug delivery, flow cytometry and cytological probing.

The high affinity of biotin for streptavidin or avidin provides the basis for many established procedures for the detection and isolation of biotin-associated targets. The binding between avidin and biotin (affinity constant, k approx. 10-15 M) is regarded as one of the strongest non-covalent, biological interactions, know. (N. M. Green, Methods Enzymol. 184:51-67,1990). This strong binding is maintained even when either or both binding partners are bound covalently to other materials. The bond forms very rapidly and is considered to be stable under a wide range of pH, temperature and other denaturing conditions (Savage et al., Avidin-Biotin Chemistry: A Handbook, 1992: 1-23, Rockford, Pierce Chemical Company). This extraordinary affinity, coupled with the ability of biotin and its derivatives to be incorporated easily into various biological materials, endows streptavidin-biotin systems with great versatility.

Dissociation of biotin from streptavidin is reported to be about 30 times faster than dissociation of biotin from avidin. (Piran & Riordan. J. Immunol. Methods 133, 141-143, 1990). The two molecules differ also with respect to non-specific binding. Avidin is glycosylated and the presence of sugar moieties cause it to bind non-specifically to biological materials. These non-specific interactions make avidin less suitable than streptavidin for many applications. Deglycosylated avidin is commercially available from Southern Biotechnology Assoc. Inc., Birmingham, AL, USA and from Accurate Chemical & Scientific Co., Westbury, NY, USA) under the trade mark NEUTRALITE AVIDIN™.

Most applications which use the biotin-streptavidin (or avidin) linkage rely on the essentially irreversible binding of the two binding partners. There are, however, many cases in which release of bound biotin is desirable, e. g. during the recovery of cells using biotinylated antibodies.

A number of strategies to disrupt or reverse the biotin-streptavidin linkage have been reported (Lee & Vacquier, Anal Biochem. 206: 206-207, 1992, Elgar & Schofield, DNA Sequence 2: 219-226,1992, and Conrad & Krupp, Nucleic Acids Res. 20: 6423-6424,1992).

The high affinity necessitates the use of harsh chemical reagents and complex procedures, e. g. boiling in high salt conditions or use of formamide and EDTA heated to 94 °C for several minutes (Tong & Smith, Anal. Chem. 64: 2672-2677, 1992), or 6 molar guanidine HCl, pH 1.5 to achieve partial or complete bond disruption. U.S. Patent No. 5,387,505 describes a method for the separation of a complex comprising a biotinylated target nucleic acid and avidin-coated polymeric particles. This method involves heating of the complex to temperatures of at least 65 °C, in the presence of a salt wash solution comprising sodium chloride, SDS and EDTA. In WO 02/061428 the inventor describe a method of disrupting a biotin-streptavidin or biotin-avidin linkage, by incubating the conjugate with an effective amount of purified or distilled water at a temperature of about 80 °C in the absence of additional processing steps. Such conditions are generally harmful to any bound moiety, but they work for nucleic acids as the upper temperature where the nucleic acids are damaged is about 300 °C. The use of such conditions to reverse the biotin-streptavidin linkage is therefore generally undesirable, especially in the purification of proteins or separation of cells, bacteria and viruses etc. when it is important to preserving the cells integrity and maintain viability or infectivity.

A number of methods have been developed in an attempt to create a releasable streptavidin-biotin or avidin-biotin conjugate.

Some scientists have introduced chemical cleavable linkers to link the biotin to one binding partner. Shimkus et al (Proc. Natl. Acad. Sci. USA, 82, pp. 2593-2597, 1985) describe the use of a disulfide bond in a linker that joined biotin to C-5 of the pyrimidine ring, as a means for reversibly binding nucleotides to avidin-agarose columns. This principle is also described in U.S. Patent No. 4,772,691.

U.S. Patent No. 5,215,927 suggests the insertion of linkages that are specifically cleavable by enzymatic or chemical agents between the biotin molecule and the specific binding reagent, e.g. peptide bonds cleavable by various peptidases, disaccharide linkages cleavable by disaccharidases, or chemical bonds that can be selectively broken under mild reducing, oxidizing, acidic, or basic conditions.

One such linker, Sulfo-NHS-SS-biotin, is commercially available (Pierce Biotechnology Inc, Rockford, IL. USA). It is an amine-reactive biotinylation reagent that contains an extended spacer arm to reduce steric hindrances associated with avidin binding. Using this group, Sulfo-NHS-SS-biotin is linked to a target molecule and the biotin portion removed by thiol cleavage using 50mM DTT. This complex approach is slow and of limited use since thiols normally disrupt native protein disulfide bonds.

One method used to dissociate the streptavidin-biotin bond involves proteinase K digestion of streptavidin (Wilchek & Bayer, Anal. Biochem. 171:1-32, 1988). Proteinase K is useful only when the biotinylated product does not comprise proteins.

The commercial available CELLection™ Biotin Binder (Dynal Biotech AS, Prod. No. 115.33) contains magnetic beads coated with streptavidin via a DNA linker to provide for cleavable site for target release. The release occurs by incubation with DNAse for 15 minutes at room temperature, followed by vigorous pipetting to maximize cell release.

The use of enzymatic or chemical cleavable linkers are sub-optimal for the isolation of truly native cells from biological environments.

Other methods include the use of monomeric avidin (Pierce Biotechnology Inc, Rockford, IL. USA.), cleavage of the biotin or streptavidin and the use of biotin analogues like N-hydroxysuccinimide-iminobiotin and amidobiotins.

N-hydroxysuccinimide-iminobiotin (NHS-iminobiotin) is a guanido analog of NHS-biotin with a pH sensitive binding affinity for streptavidin. The drawback to this system is that binding requires a pH of 9.5 or above, while the complete dissociation of NHS-iminobiotin from streptavidin requires a pH of less than 4. Thus, the use of NHS-iminobiotin is not suited for isolation of native cells.

Others have reported various approaches to disrupt the biotin-streptavidin complex under more mild conditions, such as introducing photocleavable biotin phosphoramidites (Olejnik et al., Nucleic Acids Res. 24: 361-366, 1996) or the use of polymer conjugates together with streptavidin mutants that yields temperature or pH dependent release. For example, Ding et al.(Bioconjugate Chem. 10: 395-400,1999) have conjugated a temperature-sensitive polymer, poly(N-isopropylacrylamide)(NIPAAm), to a genetically engineered streptavidin(SAv) to produce a conjugate capable of binding biotin at room temperature or lower and releasing bound biotin at 37 °C. More recently, the same group conjugated a pH sensitive polymer (a copolymer of NIPAAm and acrylic acid) to the same specific site on the genetically engineered SAv molecule (Bioconjugate Chem. 11:78-83, 2000). Lowering the pH was found to cause the polymer to collapse leading to blockage of biotin binding, whereas raising the pH caused the polymer to fully hydrate thereby permitting biotin to bind.

U.S. Patent No. 5,332,679 describes immunoassays or DNA probe assays utilizing a reversible binding displacement system based on biotin and streptavidin. In the assay, a releasable ligand, a binding partner for the releasable ligand, an analyte of interest, an analytically detectable (reporter) group, and at least one binding partner for the analyte, are first attached to an insoluble phase so as to form reporter-labeled complex bound to an insoluble phase, followed by the addition of a displacer ligand which displaces the releasable ligand along with some portion of the reporter-labeled complex, so that the released reporter is analytically detectable in a free liquid medium and can be related to the concentration of analyte in the sample. For example, where the releasable ligand is dethiobiotin and the binding partner is streptavidin, biotin, which has a higher affinity constant for streptavidin than does dethiobiotin, can be used as the displacer ligand. Examples of suitable displacer ligands include biotin, dethiobiotin, streptavidin, or avidin. Biotin is a preferred displacer ligand. Examples of suitable releasable ligands include dethiobiotin, iminobiotin, and functionalized azo dyes, streptavidin, succinylated avidin, and avidin. Dethiobiotin is preferred.

The family of patents U.S. Patent No. 4,656,252; 4,478,914; and 4,282,287 describes process of preparing a multiple-layer system involving successive, repetitive attachment of specific, molecular or particulate layers of a proteinaceous material and ligand material, to form a multiple-layer system. The proteinaceous material comprises proteins such as avidin and the ligand material comprises proteins such as biotin and derivatives, analogues or substitutes of these. Typical biotin analogues are desthiobiotin and biotin sulfone, and typical biotin derivatives include, caproylamidobiotin and biocytin.

During its formation, the multiple-layer system is attached (covalently or non-covalently) to a solid surface. The system and process may be used to change or modify properties of a solid surface including the extent of attachment of molecules to a surface for enhancing immunoassay and affinity chromatography.

The amidobiotin compounds described comprise biotin, biotin derivatives, biotin analogues, and biotin substitutes having a reactive carboxylic group covalently bonded through an amido group to an amino carboxylic acid and the carboxylic group of the amino acid covalently reacted to a hydroxy group of a cyclic compound such as N-hydroxysuccinimide (NHS) or to the reactive amino group of macromolecules such as enzymes. The general formula of the amidobiotin compounds of the invention comprise: Biotin-C(=O)NHRC(=O)(O)ₘN(H)ₙY where R is a spacer group to separate the biotin from the macromolecule and potential steric hindrance. Typically R may be an aryl group such as a phenylene group or alkyl substituted phenylene radical, an alicyclic group such as a C5 or C6 group or preferably an alkyl group such as a C1 - C12 or more such as a C3 -C10 polymethylene group. Y is a reactive amino or hydroxyamino compound such as a proteinaceous material containing reactive amino groups, e.g. enzymes. The integer n or m may be 0 or 1 depending on whether the amino reactant Y is a primary or secondary or hydroxy amine. Typical novel compounds would comprise benzoyl amidobiotin-N-hydroxysuccinimide; C1-C12 alkanoyl amidobiotin-N-hydroxysuccinimide; for example, caproylamidobiotin-NHS; and enzyme conjugates thereof. U.S. Patent No. 4,656,252 describes three different amidobiotines.

Such modified biotin derivatives are commercially available under the name DSB-X™ Biotin (Molecular Probes, Eugene, OR, USA). DSB-X™ biotin is a derivative of desthiobiotin, a stable biotin precursor. DSB-X™ biotin utilizes a seven-atom spacer to increase the ability of the DSB-X™ biotin conjugate to bind in the deep biotin-binding pocket of streptavidin or avidin. The derivative has a moderate affinity for avidin and streptavidin. Their interaction is rapidly reversed by low concentrations of free biotin or desthiobiotin at neutral pH and room temperature. Targets complexed with DSB-X biotin―labeled molecules can be selectively detected with avidin or streptavidin conjugates or isolated on affinity matrices followed release of DSB-X biotin―labeled biomolecules, under gentle conditions (Hirsch et al. Anal. Biochem. 308, 343-357, 2002). In their publication Hirsch et al describes several reversible systems. In a multiwell plate assay displacement takes place using 5 mM free biotin and incubation either for 2 hours at room temperature or overnight at 4 °C. For columns they recommend elution with > 25 mM displacement ligand in 10 to 20 column volumes, and for dot-blots they talk about 2-16 hr incubation with displacement ligand. DES-X™ biotin can be conjugated to various molecules, with the use of streptavidin or avidin coated solid phase for immobilization of target, or one may use a DES-X™-coated solid phase and streptavidin or avidin labeled molecules. Molecular Probes provides a variety of antibody conjugates of DSB-X™ biotin as well as DSB-X™ biotin agarose.

Another method to reduce the affinity of biotin to streptavidin or avidin is the used of recombinant or chemically modified streptavidin or avidin. WO 01/05977 of Kulomaa et al. covers mutations in both avidin and streptavidin by replacing a specific tryptophan residue with lysine. Both of these mutant proteins produce a stable dimer. These stable dimers exhibit reversible biotin-binding properties when tested with 0.5 mM biotin in buffer (0.5% BSA; 0.5% Tween 20 and 1 M NaCl in PBS) at 37 °C for 1 hr.

U.S. Patent No. 6,022,951 also describes a mutated recombinant streptavidin with reduced affinity for biotin. The inventors suggested modifications to streptavidin in the form of one or more deletions, insertions, point mutations or combinations of these genetic alterations that alter, but maintain the biotin-binding site. In order to disrupt the streptavidin-biotin bond of their mutated streptavidin they can use between 0.1 mM to 10 mM of biotin. In addition, elution may be performed in a high or a low pH, in high salt, or in the presence of ionic detergents, dissociating agents, chaotropic agents, organic solvents, protease (protease K) or water. In their Example 11 they used their reduced affinity streptavidin coated onto glass-beads for the isolation and separation of B and T cells. They used a 3 ml column containing 2 ml of glass-beads coated with anti-human IgG. 1 ml of lymphocytes were added to the column at a flow-rate of 500 µl/minute and the T-cell containing flow through fraction was collected. The column was washed with an additional 15 ml of PBS at 500 µl/minute to recover additional T-cells. The bound B-cells were eluted from the column using 15 ml of 2 mM biotin in PBS. The overall time for this procedure will be at least 1 hour.

U.S. Patent Nos. 6,391,571; 6,312,916; and 6,417,331 describe muteins of avidin and streptavidin having a reduced binding affinity for biotin made using various amino acid substitutions and deletions. The inventors showed that they could elute their biotinylated Bovine Serum Albumin from their Spherosil-NH₂ Streptavidin mutein adsorber column using 50 mM ammonium acetate, pH 3.0 or/and a gradient of 9 to 10 mM iminobiotin or biotin. Their biotinylated Fab antibody fragment was eluted using PBS buffer, pH 7.2 and a gradient of 0 to 10 mM biotin.

U.S. Patent Nos. 6,165,750 and 6,156,493 describe a chimeric streptavidin tetramer having at least one monomer containing an amino acid modification that produces a reduced binding affinity for biotin, a modified off-rate, a modified on-rate, or an altered binding enthalpy. They used biotin columns and eluted their mutated streptavidin by adding 2mM Biotin to the flow buffer

U.S. Patent No. 5,168,049 describes a polypeptide which is substantially immunologically equivalent to natural streptavidin and is able to bind to biotin or biotin derivatives or analogues.

Other patents report the generation of peptides with binding activity for streptavidin. U.S. Patent No. 5,506,121 describes the generation of such peptides (Strep-tags) which can be eluted from streptavidin agarose columns using a solution of 1 mM iminobiotin or 5 mM lipoic acid. The advantage of such peptide ligands compared to biotin is essentially that their coding sequence is linked at the DNA level with the gene of a desired protein and can subsequently be co-expressed together with that of the protein by which means a recombinant protein labeled with the peptide ligand is formed. U.S. Patent No. 6,103,493 offers streptavidin muteins whose binding affinity for peptide ligands is such that they can be competitively eluted by other streptavidin ligands e.g. biotin, iminobiotin, lipoic acid, desthiobiotin, diaminobiotin, HABA (hydroxyazobenzene-benzoic acid) or/and dimethyl-HABA. The bound protein can be eluted step-wise by applying 10 ml each of diaminobiotin, desthio-biotin and biotin at a concentration of 2.5 mM.

U.S. Patent No. 5,973,124 describes a method of modifying avidin-type molecules. The essential tyrosine residue in the biotin-binding site is modified in such a way that its pKa is decreased in comparison to the pKa of the unmodified tyrosine residue in the corresponding unmodified avidin-type molecule. The modification is achieved by substitution at one or both ortho positions to the hydroxy radical of the tyrosine residue by radicals such as nitro, halogen, azo and amino. They suggest that such modification can be done on different avidin-type molecules including: (i) native egg-white avidin; (ii) recombinant avidin; (iii) deglycosylated forms of avidin; (iv) bacterial streptavidin; (v) recombinant streptavidin; (vi) truncated streptavidin; and derivatives thereof. The inventors showed they could release a biotinylated compound from a column containing a modified avidin-type molecule by addition of either alkaline solutions (e.g. pH 10) or by adding excess biotin (e.g. 0.6 mM at any pH). Based on their figures they need about 5 column volumes of biotin-containing buffer to achieve the release (Morag et al. Anal. Biochem. 243, 257-263, 1996.

Such nitrated avidin derivatives are commercially available from Molecular Probes, Eugene, OR, USA, under the name CaptAvidin®

Despite advances made to date, there still exists a need for new and improved methods for selectively isolating and releasing cells and other biomolecules, None of the previously reported methods for the reversible binding between biotin and streptavidin are optimal for use for the isolation of conformation sensitive targets or native cells. Consequently, there is a continuing need in the art for alternative methods for reversibly and reliably disrupting the binding between biotin and streptavidin (or avidin).

### SUMMARY OF THE INVENTION

Aspects of the present invention provide a simple method of reversal of the binding between a biotinylated moiety and a biotin-binding compound like streptavidin (or avidin) without destroying the native conformation of the target or the native status of the isolated cells. The strong interaction between streptavidin or avidin-biotin can be made much weaker by using a combination of nitro-streptavidin or avidin and modified biotin like desthiobiotin or a derivative thereof like DSB-X Biotin. A protein, such as an antibody directed against a cell-surface antigen may be biotinylated with the modified biotin. This antibody is captured or bound to the nitro-streptavidin or avidin which again is immobilized on a solid surface. This solid-phase/antibody complex is used to capture specific cells from a complex mixture. The cells with the bound antibody is then released under very gentle and fast conditions by contacting the complex with an effective amount of free biotin. The liberated cells with the biotinylated antibodies can thereby be isolated and purified.

A novel aspect of the present method over the prior art is that both parts of the biotin-streptavidin binding pair is modified to have substantially reduced affinity for each other and, as a result, the speed of release becomes much faster than what is reported in the prior art. The method avoids the harsh chemical denaturing conditions that have been previously used. It also avoids any lengthy incubation with displacement ligand, or the dilution effect occurring when eluting isolated target from affinity columns. Isolated cells will be closer to their native state than if isolated using previously available methods.

The method has been shown to work very well for cell isolation and detachment procedures. The method can be used in various other procedures such as detection, identification, determination, purification, separation and/or isolation of target proteins or nucleic acid molecules. Kits for carrying out the method of the invention are also provided.

### DESCRIPTION OF THE FIGURES

The following figures form part of the present specification and are included to further demonstrate certain aspects of the present invention. The invention may be better understood by reference to one or more of these figures in combination with the detailed description of specific embodiments presented herein.

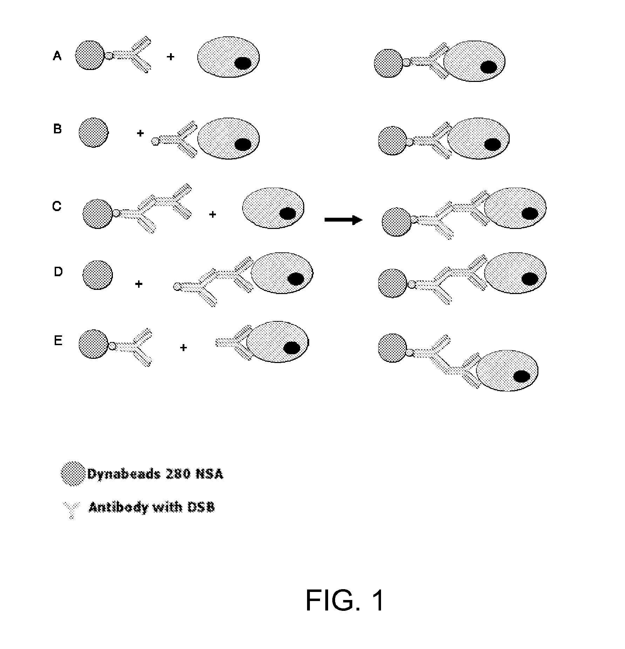

FIG. 1 is a schematic presentation of five different principles for labeling and isolation of specific cells. The two principles where one primary antibody accounts for the labeling and cell isolation are shown in panel A and B, whereas the dual labeling principles are shown in panels C, D and E, where both a primary and a secondary antibody are used.

FIG. 1A shows a direct one-layer labeling technique where the specific antibodies are labeled with modified biotin and bound to the modified streptavidin-coated solid support prior to incubation with target cells.

Fig. 1B shows an indirect one-layer labeling technique where the specific antibodies are labeled with modified biotin and incubation with target cells prior to incubation with the modified streptavidin-coated solid support.

Fig. 1C shows a direct two-layer labeling technique where the antibodies directed against the target antibodies are labeled with modified biotin and bound to the modified streptavidin-coated solid support prior to incubation with anti-target antibodies. These complexes are then incubated with target cells.

Fig. 1D shows an indirect two-layer labeling technique where the complex of biotin-labeled anti-antibodies and anti-target antibodies are incubated with target cells prior to incubation with the modified streptavidin-coated solid support.

Fig. IE, shows a combined two-layer labeling technique where the modified biotin-labeled anti-antibodies are bound to the modified streptavidin prior to incubation with anti-target antibodies already bound to target cells.

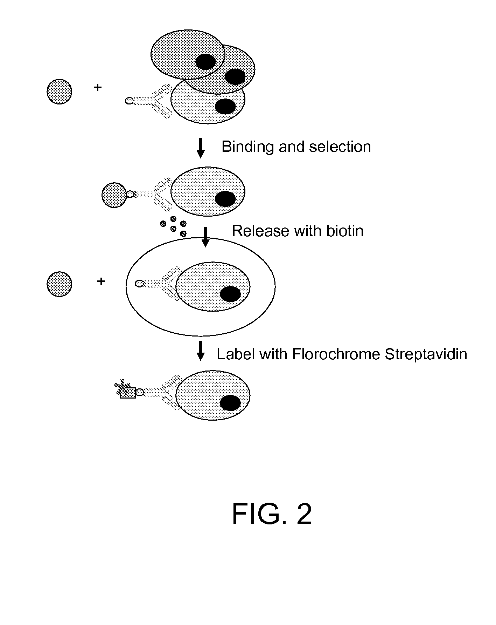

FIG. 2 is a is a schematic presentation of the method of use of the present invention showing how the modified biotinylated cell-bound antibody can be used for binding with fluorochrome labeled streptavidin for detection in fluorescence microscopy or flow-cytometry.

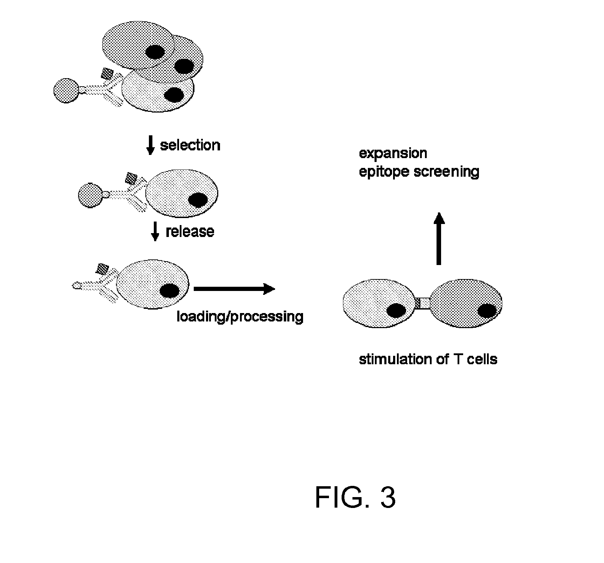

FIG. 3 is a schematic presentation of the method of use of the present invention for the specific stimulation of T cells via antigen presenting cells (APC). Shown is the DSB-labeled modified antibody with an antigenic peptide inserted in the structure either genetically or chemically (shown as a square). After APC isolation the retained modified antibody will be internalized and processed (loading/processing). The subsequent presentation of the antigenic peptides by the APCs can be used for stimulation of specific T-cells. The T-cells will recognize the peptide via their T-cell receptors (TcR) in the context of Major Histo-compatibility Complex (MHC)class II.

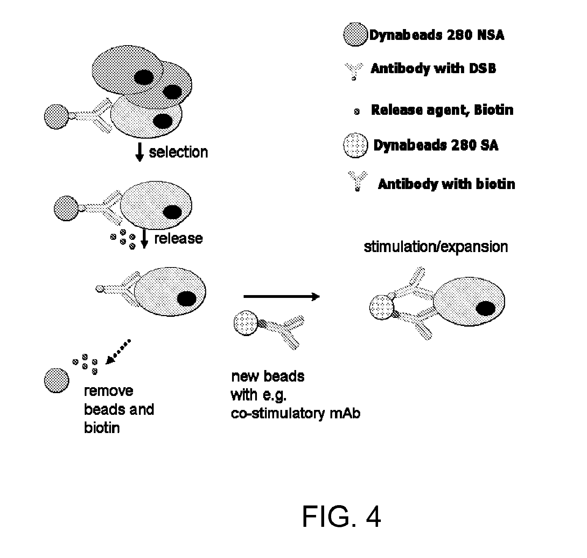

FIG. 4 is a schematic presentation of the method of use of the present invention showing how isolated cells can be stimulated via the antibody used for cell isolation. Isolated cells will retain the DSB-linked-antibody used for isolation on the cell surface. Introduction of additional biotinylated co-stimulatory molecules and streptavidin coated Dynabeads will create a cell-bead complex pushing cells into activation, proliferation and/or differentiation.

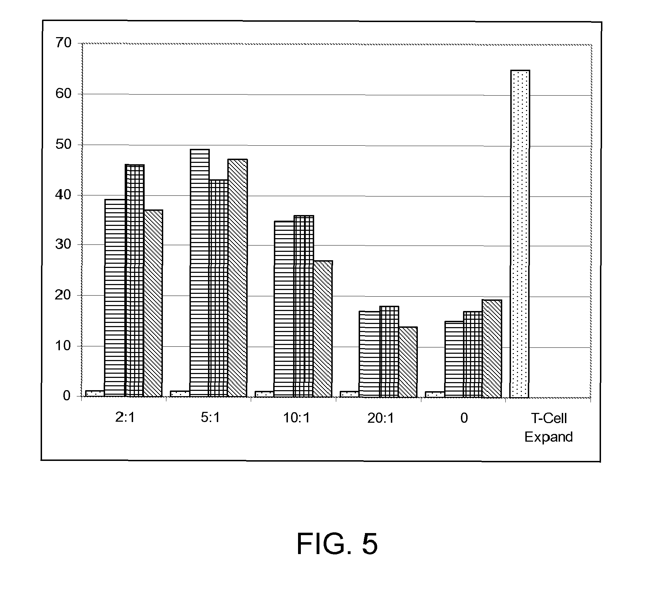

FIG. 5 is a graph showing proliferation of CD3⁺ T cells after stimulation with Dynabeads M-280 streptavidin, biotin-anti-CD28 and DSB-linked-anti-CD3. CD3⁺ T cells were isolation using DSB-linked-anti-CD3 and Nitro-Streptavidin-Beads. After release cells retained the DSB-linked-anti-CD3 on the cell surface, and biotin-anti-CD28 and Dynabeads M-280 Streptavidin were introduced to generate the stimulatory signals (through the TCR/CD3 complex and co-stimulatory molecule) needed for the cells to enter cell cycle. Cells were pulsed with ³H-thymidin after 48 hours of stimulation and harvested after 72 hours, as described in Example 4.

### DETAILED DESCRIPTION OF THE INVENTION

While compositions and methods are described in terms of "comprising" various components or steps (interpreted as meaning "including, but not limited to"), the compositions and methods can also "consist essentially of" or "consist of" the various components and steps, such terminology should be interpreted as defining essentially closed-member groups.

Methods

One embodiment of the present invention provides a process for the recovery of the biotinylated ligand in a method employing the avidin-biotin technology (as shown in FIG. 1A), which comprises the following steps:

(a) immobilize a modified biotinylated ligand onto a modified streptavidin-type molecule attached to a solid support;

(b) carry out the desired reaction or separation process between the thus immobilized modified biotinylated ligand and its target;

(c) remove the modified biotinylated ligand/target complex from the immobilized modified streptavidin by the addition of low concentrations of free biotin; and

(d) recover the modified biotinylated ligand/target complex.

An alternative embodiment of the invention is directed towards a method comprising the following steps (as illustrated FIG. 1B):

(a) incubate the modified biotinylated ligand and its target;

(b) immobilize the modified biotinylated ligand/target complex onto a modified streptavidin-type molecule attached to a solid support;

(c) remove the modified biotinylated ligand/target complex from the immobilized modified streptavidin by the addition of low concentrations of free biotin; and

(d) recover the modified biotinylated ligand/target complex.

A third embodiment of the invention is directed towards a method comprising the following steps (as illustrated in FIG. 1 C):

(a) immobilize a modified biotinylated anti-ligand onto a modified streptavidin-type molecule attached to a solid support;

(b) incubate the complex from step a with a ligand;

(c) carry out the desired reaction or separation process between the thus immobilized complex of step b with its target;

(d) remove the modified biotinylated anti-ligand/ligand/target complex from the immobilized modified streptavidin by the addition of low concentrations of free biotin; and

(e) recover the modified biotinylated anti-ligand/ ligand/target complex.

A fourth embodiment of the invention is directed towards a method comprising the following steps as illustrated in FIG. 1D):

(a) incubate the modified biotinylated anti-ligand with the ligand;

(b) incubate the complex from step a, with the target;

(c) immobilize the complex from step b onto a modified streptavidin-type molecule attached to a solid support;

(d) remove the modified biotinylated anti-ligand/ ligand/target complex from the immobilized modified streptavidin by the addition of low concentrations of free biotin; and

(e) recover the modified biotinylated anti-ligand/ ligand/target complex.

A fifth embodiment of the invention is directed towards a method comprising the following steps as illustrated in FIG. 1E):

(a) immobilize a modified biotinylated anti-ligand onto a modified streptavidin-type molecule attached to a solid support;

(b) incubate the complex from step a with a ligand;

(c) carrying out the desired reaction or separation process between the thus immobilized complex of step b with its target;

(d) removing the modified biotinylated anti-ligand/ ligand/target complex from the immobilized modified streptavidin by the addition of low concentrations of free biotin; and

(e) recovering the modified biotinylated anti-ligand/ ligand/target complex.

An advantage of the described method over the prior art is that the release of the bound cells is much faster and performed under very gentle conditions such as about 50 µM to about 500 µM Biotin in PBS buffer or cell media at physiological pH.

The terms "biotin" as used herein are intended to refer to biotin (cishexahydro-2oxo-1H-thieno[3,4]imidazole-4-pentanoic acid) and any biotin derivatives and analogs. Such derivatives and analogues are substances which form a complex with the biotin binding pocket of native or modified streptavidin or avidin. Such compounds include, for example, iminobiotin, desthiobiotin and streptavidin affinity peptides, and also include biotin-.epsilon.-N-lysine, biocytin hydrazide, amino or sulfhydryl derivatives of 2-iminobiotin and biotinyl-ε-aminocaproic acid-N-hydroxysuccinimide ester, sulfo-succinimide-iminobiotin, biotinbromoacetylhydrazide, p-diazobenzoyl biocytin, 3-(N-maleimidopropionyl) biocytin. A preferred derivative of biotin to be used in the present invention is desthiobiotin or its derivative DSB-X Biotin, commercially available from Molecular Probes, Eugene, OR, USA; product number D20658)

The term "biotinylated substances" or "moieties" is to be understood as conjugates of modified biotin or biotin analogues with other moieties such as biomolecules, e. g. nucleic acid molecules (including single or double stranded DNA, RNA, DNA/RNA chimeric molecules, nucleic acid analogs and any molecule which contains or incorporates a nucleotide sequence, e. g. a peptide nucleic acid (PNA) or any modification thereof), proteins (including glycoproteins, enzymes, peptides library or display products and antibodies or derivatives thereof), peptides, carbohydrates or polysaccharides, lipids, etc., wherein the other moieties are covalently linked to the modified biotin or biotin analogues. Many biotinylated ligands are commercially available or can be prepared by standard methods. Processes for coupling a biomolecule, e. g. a nucleic acid molecule or a protein molecule, to biotin are well known in the art (Bayer and Wilchek, Methods in Molec. Biology 10, 143. 1992).

The term "binding partner" is defined as any biological or other organic molecule capable of specific or non-specific binding or interaction with another biological molecule, which binding or interaction may be referred to as "ligand" binding or interaction and is exemplified by, but not limited to, antibody/antigen, antibody/hapten, enzyme/substrate, enzyme/inhibitor, enzyme/cofactor, binding protein/substrate, carrier protein/substrate, lectin/carbohydrate, receptor/hormone, receptor/effector or repressor/inducer bindings or interactions. The appropriate ligands will be chosen depending on the use to which the method of the invention is desired to be put.

Other specific affinity adsorbent moieties, such as wheat germ agglutinant, anti-idiotypic antibodies and dye ligands may also be coupled to the modified biotin to isolate glycosylated proteins such as SP1 transcription factor, dye binding proteins such as pyruvate kinase and liver alcohol dehydrogenase, and other antibodies.

In some instances, the ligand is an antibody which is directed against a drug, hormone, antibiotic or other compound having antigenic properties. The antibody may also be directed against another antibody (that is, an anti-antibody). Both monoclonal and polyclonal antibodies can be used, and they can be whole molecules or various fragments thereof. Antibody specific for a particular ligand may be produced by methods well known and documented in the art.

Antibodies for use in methods of the present invention may be of any species, class or subtype providing that such antibodies are capable of forming a linkage with a particular target ligand and can be biotinylated with a modified biotin. Thus antibodies for use in the present invention include:

any of the various classes or sub-classes of immunoglobulin, e. g. IgG, IgA, IgM, IgD or IgE derived from any animal e. g. any of the animals conventionally used, e. g. sheep, rabbits, goats, or mice,

monoclonal antibodies

intact antibodies or "fragments" of antibodies, monoclonal or polyclonal, the fragments being those which contain the binding region of the antibody, e. g. fragments devoid of the Fc portion (e.g. Fab, Fab', F(ab')2, Fv), the so called "half molecule" fragments obtained by reductive cleavage of the disulphide bonds connecting the heavy chain components in the intact antibody.

antibodies produced or modified by recombinant DNA or other synthetic techniques, including monoclonal antibodies, fragments of antibodies, "humanized antibodies", chimeric antibodies, or synthetically made or altered antibody-like structures. Also included are functional derivatives or "equivalents" of antibodies e. g. single chain antibodies.

Alternatively, the ligand can be an antigenic material (including mono- or multivalent or multideterminant antigens).

The terms "conjugate" and "complex" as used herein refer to any conjugate or complex comprising a biotin compound and a biotin-binding compound, in which the biotin compound and biotin-binding compound are linked by non-covalent bonding. Typically, biotin will be bound or linked to one or more, preferably one, biological or chemical entity, e. g. a biomolecule. As explained above, such biotin compounds containing biotin linked to other entities are also referred to herein as "biotinylated moieties".

The term "biotin-binding" compound as used herein is intended to encompass any compound which is capable of tightly but non-covalently binding to biotin or any biotin compound. Preferred biotin-binding compounds include modified streptavidin and avidin, as well as derivatives and analogues thereof e.g. nitro-streptavidin.

The term "avidin" as used herein refers to the native egg-white glycoprotein avidin as well as derivatives or equivalents thereof, such as deglycosylated or recombinant forms of avidin, for example, N-acyl avidins, e.g., N-acetyl, N-phthalyl and N-succinyl avidin, and the commercial products ExtrAvidin, Neutralite Avidin and CaptAvidin

The term "Streptavidin" as used herein refers to bacterial streptavidins produced by selected strains of Streptomyces, e.g., Streptomyces avidinii, as well as derivatives or equivalents thereof such as recombinant and truncated streptavidin, such as, for example, "core" streptavidin.

Certain Avidin/Streptavidin materials are commercially available, e.g. native avidin and streptavidin, non-glycosylated avidins, N-acyl avidins and truncated streptavidin, or can be prepared by well-known methods (see Avidin-biotin technology, Methods of Enzymology, Vol. 184: 1-671, 1990. In that reference Green, describe preparation of avidin and streptavidin; Hiller et al., the preparation of non-glycosylated avidin; Bayer et al., the preparation of streptavidin and truncated streptavidin, Chandra & Gray describe recombinant avidin). Both native and recombinant forms of streptavidin and avidin may be used in the methods described herein as long as they can be modified as described in US-A-5,973,124. A preferred derivative of streptavidin to be used in the present invention is Nitro-streptavidin, prepared as described in Example 2. A preferred derivative to use as starting material is recombinant core-streptavidin.

In a preferred embodiment of the invention either the biotin-analogue or the modified biotin-binding compound, is immobilized on an immobilizing moiety, e. g. a solid support. Preferably, the modified biotin-binding compound, e. g. nitro-streptavidin (or avidin), will be immobilized. The attachment of either component of the linkage to a solid phase allows easy manipulation of the linked components. Thus, the attachment to some kind of solid phase can enable the separation of the linked components from the rest of the components in the mixture. This can be achieved for example by carrying out washing steps, or if the components are attached to magnetic beads, using a magnetic field to effect physical separation of the linked component from the rest of the components in the mixture.

The solid support may be any of the well-known supports or matrices which are currently widely used or proposed for immobilization, separation etc., in chemical or biochemical procedures. These may take the form of particles, sheets, dip-sticks, gels, filters, membranes, microfibre strips, tubes, wells or plates, fibres or capillaries, combs, pipette tips, microarrays or chips or combinations thereof, and conveniently may be made of a polymeric material, e. g. agarose, Sepharose, cellulose, nitrocellulose, alginate, Teflon, latex, acrylamide, nylon membranes, plastic, polystyrene, glass or silica or metals. Biochips may be used as solid supports to provide miniature experimental systems as described for example in Nilsson et al. (Anal. Biochem.224: 400-408, 1995) or as a diagnostic tool. Numerous suitable solid supports are commercially available.

Preferred solid supports are materials presenting a high surface area for binding of the modified biotin or modified biotin-binding compound. Such supports will generally have an irregular surface and may for example be porous or particulate, e. g. particles, fibers, webs, sinters or sieves. Particulate materials e. g. beads are generally preferred due to their greater binding capacity, particularly polymeric beads/particles.

Conveniently, a particulate solid support used according to the invention will comprise spherical beads. The size of the beads is not critical, but they may for example be of the order of diameter of at least 0.01 µm, and have a maximum diameter of preferably not more than 10 and more preferably not more than 6 µm. For example, beads of diameter 1.0 µm, 2.8 µm and 4.5 µm have been shown to work well.

Monodisperse particles, that is those which are substantially uniform in size (e. g. size having a diameter standard deviation of less than 5%) have the advantage that they provide very uniform reproducibility of reaction. Monodisperse polymer particles produced by the technique described in U.S. Patent No. 4,336,173 are especially suitable.

The particles can be composed of the same polymer throughout, or they can be core-shell polymers as described, for example, in U.S. Patent No. 4,703,018 and EP-A-0280556 where the shell polymer has the requisite reactive groups.

Non-magnetic polymer beads suitable for use in the method of the invention are available from Dynal Biotech AS (Oslo, Norway) under the trademark DYNOSPHERES, as well as from Qiagen, GE Healthcare Life Sciences, Serotec, Seradyne, Merck, Nippon Paint, Chemagen, Promega, Prolabo, Polysciences, Agowa and Bangs Laboratories.

However, to aid manipulation and separation of immobilized material, and also to facilitate automation if required, magnetizable ("magnetic") beads are preferred. The term "magnetic" as used herein means that the support is capable of having a magnetic moment imparted to it when placed in a magnetic field, and thus is displaceable under the action of that field. In other words, a support comprising magnetic particles may readily be removed from other components of a sample by magnetic aggregation, which provides a quick, simple and efficient way of separating the particles following the binding of any modified biotin or modified biotinylated moieties. In addition, such magnetic aggregation is a far less rigorous method of separation than traditional techniques such as centrifugation which generate shear forces which may disrupt cells or degrade any other moieties, e. g. proteins or nucleic acids attached to the modified biotin molecules.

Thus, the magnetic particles with the modified biotin or modified biotinylated moieties attached via conjugation to a modified biotin-binding compound, e. g. nitro-streptavidin (avidin), may be removed onto a suitable surface by application of a magnetic field, e.g. using a permanent magnet. It is usually sufficient to apply a magnet to the side of the vessel containing the sample mixture to aggregate the particles to the wall of the vessel and to remove the remainder of the sample so that the remaining sample and/or the particles are available for any desired further steps.

Alternatively, the method may be performed using an automated system for handling of such magnetic particles. The sample containing the target cells may be transferred to such an apparatus, and magnetic particles carrying antibodies against target, linked though a modified biotin/nitrostreptavidin complex, can be added. The isolated support-bound cells may be washed if desired, and transferred to other vials containing the displacement biotin, followed by removal of the released particles. Particular mention may be made in this regard of the Bead Retriever, available from Dynal Biotech AS, Norway. The apparatus has a system for ready and efficient transfer of the support (carrying cells) from one well to another.

Preferably such magnetic particles are superparamagnetic to avoid magnetic remanence and hence clumping, and advantageously are monodisperse (i. e. are substantially uniform in size, e. g. size having a diameter standard deviation of less than 5%) to provide uniform kinetics and separation. The preparation of superparamagnetic monodisperse particles is described by Sintef in EP-A-106873.

The well-known monodisperse polymeric superparamagnetic beads sold by Dynal Biotech AS (Oslo, Norway) under the trade mark DYNABEADS, are exemplary of commercially available magnetic particles which may be used or modified for use according to the invention.

The modified biotin-binding compound (e.g. nitro-streptavidin or avidin), or the modified biotin, if desired, may be covalently attached to a suitable support through reactive groups on the substrate surface by methods well known in the art. These include, for example, attachment through hydroxyl, carboxyl, aldehyde or amino groups which may be provided by treating the immobilizing support to provide suitable surface coating.

Alternatively, supports with functionalized surfaces are commercially available from many manufacturers, such as those particle manufacturers described above.

Magnetic particles with the following functionalized surfaces are available from Dynal Biotech AS, Oslo, Norway:

Hydrophobic beads

Dynabeads® M-450 Epoxy (with epoxy groups)

Dynabeads® M-450 Tosylactivated (with tosyl groups)

Dynabeads® M-280 Tosylactivated (with tosyl groups)

Dynabeads® MyOne™ Tosylactivated (with tosyl groups)

Dynabeads® M-500 Subcellular (with tosyl groups)

Hydrophilic beads

Dynabeads® M-270 Epoxy (with epoxy groups)

Dynabeads® M-270 Carboxylic acid (with carboxylic acid groups)

Dynabeads® MyOne Carboxylic acid (with carboxylic acid groups)

Dynabeads M-270 Amine (with amino groups).

The appropriate choice of surface may depend on the type of moieties which are attached to the modified biotin or the modified biotin-binding compound in the particular method concerned. The attachment can be achieved through amino or sulfhydryl groups of the biotin-binding compound which are available for reaction directly with reactive groups on the outer surface of the particles.

There are many useful reactive groups which react with a free amine group of the modified biotin-binding molecule. Such groups include, but are not limited to, carboxy, active halogen, activated 2-substituted ethylsulfonyl, activated 2-substituted ethylcarbonyl, active ester, vinylsulfonyl, vinylcarbonyl, aldehyde, epoxy, amino and sulfhydryl, all of which are known in the art. Some of these groups will react directly with the modified biotin-binding molecule while others, such as carboxy, require the use of a compound to produce an intermediate which will react with the modified biotin-binding compound molecule. Reagents suitable for cross-linking of the solid surface and the biotin-binding compound include cyanogen bromide, carbonyldiimidazole, glutaraldehyde, hydroxysuccinimide and tosyl chloride. Both Tosyl- and epoxy surfaces have been found to work well with the present invention.

The general procedure for preparing the reagent of this invention includes covalently attaching the modified streptavidin or avidin, or a derivative thereof to the particles using generally known reactions. Details of a representative preparatory procedure are illustrated in EXAMPLE 2 below.

The modified biotin-binding molecules of the invention described herein may be used in any method employing the avidin-biotin technology, particularly in those methods in which disruption of the binding or reversibility of the binding-complex is desirable.

The terms "reversal", "cleaving", "releasing", or "disrupting" are used herein interchangeably and are intended to mean physical separation or detachment or dissociation of the partners of the binding complex. What is required, is that the linkage between the modified biotin and the modified biotin-binding compound is disrupted or broken to allow separation of the respective entities.

The "displacement molecule" (for example, free biotin) may physically break or destabilize the linkage in a sufficient manner to allow it to be cleaved, or reversed, thus allowing the two linked entities to be separated. Furthermore, in a population of linkages, it may not be necessary for each and every linkage to be disrupted, as long as a sufficient or significant proportion are "reversed" e. g. where substantially all of the linkages are "reversed". "Substantially" in this context, may be taken to mean that at least 70% (or more preferably at least 75, 80, 85, 90 or 95%) of the linkages are reversed. Ideally, 100% of the linkages are reversed. In the linkage reversal system of the present invention, utility may be preserved even though reversal may not be 100% complete.

For example, this invention may be used in methods for the detection, identification, determination, purification, separation and/or isolation of compounds of biological interest, targets, from heterogeneous mixtures. Such compounds can be defined as any biological or chemical compound which has one or more sites for complexing with a corresponding specific ligand, and where the ligand can be biotinylated with a modified biotin.

The method of the invention may be applied to the purification or isolation of any type of cells or cellular component from any biological sample or artificial media. Representative biological samples derived from a human or animal source include whole blood, and blood-derived products such as plasma, buffy coat or leukophoresis products, serum, saliva, lymph, bile, urine, milk, faeces, cerebrospinal fluid or any other body fluids like spinal fluid, seminal fluid, lacrimal fluid, vaginal secretions, and the like, as well as stool specimens. It is also possible to assay fluid preparations of human or animal tissue such as skeletal muscle, heart, kidney, lungs, brains, bone marrow, skin and the like or cellular extracts or secretions and cell suspensions obtained by density gradient centrifugation etc., and also environmental samples such as soil, water or food samples. Such samples may be used as they are, or they may be subjected to various purification, decontamination, filtration, or concentration methods. The sample may also include relatively pure or partially purified starting materials, such as semi-pure preparations obtained by other cell or biomolecule separation processes like immunomagnetic separation.

Moreover it should be noted that the method according to the invention may be applied to the isolation and subsequent liberation of sub-cellular components such as mitochondria and nuclei, and macromolecules such as proteins and nucleic acids. The entity to be isolated may be naturally antigenic or may be made so artificially.

The target chosen may be a particular structural molecule e. g. a peptide, protein, glycoprotein, lipid or carbohydrate etc. associated with the surface of larger biological entities for example cells. Other targets may be biological substances include peptides, polypeptides, proteins, lipoproteins, glycoproteins, nucleic acids (DNA, RNA, PNA, aptamers) and nucleic acid precursors (nucleosides and nucleotides), polysaccharides, lipids such as lipid vesicles. Typical proteins which are detectable in conventional streptavidin/biotin systems, and useful herein, include cytokines, hormones, vitamins surface receptors, haptens, antigens, antibodies, enzymes, growth factors, recombinant proteins, toxins, and fragments and combinations thereof.

The term "cell" is used herein to include all prokaryotic (including archaebacteria and mycoplasma) and eukaryotic cells and other entities such as viruses and sub-cellular components such as organelles (e.g. mitochondria and nuclei). Representative "cells" thus include all types of mammalian and non-mammalian animal cells, plant cells, insect cells, fungal cells, yeast cells, protozoa, bacteria, protoplasts and viruses.

The method of the invention is particularly suited to cell isolation, particularly in the possible selection of desirable cells using antibodies directed against the cells to be isolated (as opposed to negative selection procedures where unwanted cells are removed from a cell preparation using antibodies specific for the unwanted cells).

In such method of positively isolation a desired target cell type is isolated from a mixed population of cells using solid phase coated with antibodies directed against a cell surface ligand/antigen. The antibodies carry modified biotin like DSB-X-biotin and are attached to a solid phase through the interaction with the modified streptavidin like nitro-streptavidin. The attachment to the solid phase may take place before or after binding to the target cells, whereby the solid phase and attached cells are separated from the other cells present. According to the method of the invention the solid phase-bound target cells are quickly released from the particles by the gentle addition of a displacement ligand (e. g. free biotin), to leave a positively selected population of unaffected, viable cells carrying the modified biotinylated antibodies.

One attractive aspect of the invention is that it opens up for many different post-isolation use of the cell-linked antibody/ligand. The released cells continue to carry their biotinylated antibodies. The presence of the modified biotin-group facilitates further streptavidin or avidin interactions in various research or diagnostic procedures.

By adding labeled streptavidin, for example with enzymes or with fluorescent, chemiluminescent or radioactive agents, one can detect and quantify with a high degree of sensitivity the isolated targets. Conjugated streptavidin or avidin products (with fluorescein, rhodamine, ferritin or horse radish peroxidase) are commercially available. Such labeling will facilitate the detection of the isolated cells in flow cytometry. The cells must be washed once to remove the free biotin in the release solution, then the fluorochrome-conjugated streptavidin can be added and all the isolated cells are labeled and prone to detection in fluorescence microscopy or flow-cytometry(FIG. 2).

The biotinylated antibodies attached to the released cells can also be used for the delivery of proteins into cells for *in vitro* studies of A: phagocytosis, B: delivery of toxic or metabolic substances or C: delivery of proteins/peptides for degradation and presentation by the MHC II/HLA complex on antigen presenting cells (APCs).

(a) Green fluorescent protein-labeled antibodies can be traced in endocytic compartments, or FITC conjugated streptavidin bound to the DSB-X-labeled-antibody can be used.

(b) The DSB-X-labeled-antibody can be conjugated with toxins for the study of drug delivery into cells.

(c) The DSB-X-labeled-antibody/DSB-X-protein can be directed to cell internalization and degradation/-processing and subsequent present antigenic peptides for specific T-cell stimulation. The concept of targeting APCs by antibodies for the delivery of T-cell epitopes for *in vivo* therapeutic use, has been described in U.S. Patent No. 6,294,654 of Bogen et al. and in Brekke O. H. & Sandlie I. European BioPharmaceutical Review, Spring 2002. Typical APCs are B-cells, monocytes, macrophages or dendritic cells. Any cell surface markers for such APCs can be used for targeting antibodies.

The novelty of the concept as described in aspects of the present invention is the combined *in vitro* cell isolation and *in vitro* specific T cell stimulation. When used in combination with the Dynabeads® CD3/CD28 product for T-cell expansion (Dynal Biotech AS, Oslo Norway) the specific T-cells can be even further expanded. The concept can be applied in the screening and discovery of new T-cell epitopes for vaccine development.

One can envisage direct conjugation of antigenic proteins to the antibodies instead of genetically incorporating them into the antibodies as is described in the above two reference. If the antigenic protein itself is a ligand for a cell receptor it can be directly used for cell isolation after DSB-X-conjugation (See FIG. 3).

Aspects of the present invention can also be used in connection with cell stimulation and expansion (see FIG. 4).

The cell-bound antibodies display DSB-moieties, i.e. after selection and release they are accessible for binding to Streptavidin either in solution or on a solid surface (e.g. magnetic beads). This can be used to facilitate the delivery of two or more signals to the cell. E.g. use anti-CD3 for isolation with subsequent cross linking with co-stimulatory antibodies/ligands such as anti-CD28, anti-CD137 or anti-NKG2D. This will enable a very flexible system for controlling and changing the ratio of the involved molecules (e.g. anti-CD3 ― anti-CD28 ratio).

If one wishes to have the isolated cells free of any antibody the method of the present invention can be combined with other principles of release, like that described in U.S. Patent No. 5,429,927. There the inventors describe a method of cleaving an antigen/antibody linkage by reacting the linkage with a secondary antibody which will bind to, and disrupt, the binding between the antigen and antibody thus releasing the antigen (see EXAMPLE 5).

Following release of the binding pair, i. e. following release of the modified biotinylated antibody, the nitro-streptavidin support may be re-used.

In a preferred embodiment of the invention the solid phase comprises magnetic particles and the magnetic particles and attached cells are isolated from the mixed population of cells by magnetic aggregation.

In many prior art methods to positively isolate cells using magnetic particles, to liberate cells from the particles, the cell/particle "rosettes" have been incubated overnight at 37 °C to effect separation of the cells from the particles. In some cases the cells detach from the particles, but in many cases they do not, and such poor recovery makes difficult the isolation of poorly represented cell sub-populations.

Aspects of the invention are directed to a method for the isolation of malignant cells or cell populations specific for different diseases and to characterize these cells further without interference from other contaminating cells. Also, the method may be used to isolate protective cell populations from an individual or from a group of individuals; the isolated population can then be expanded and/or potentiated before being returned to the patient under treatment. Such protective cell populations can for example be monocytes/-macrophages, lymphocytes or bone marrow stem cells.

A method can also be used for the isolation and culture of infectious agents such as bacteria or virus from a patient in order to quantify them or characterize their infectivity, toxicity or susceptibility to drug treatment. Body fluids, such as blood of a patient may be contacted with a support with antibodies specific for viral surface antigens. If the antibody was crosslinked to the solid support by a modified streptavidin, bound infectious agents may be released without harm with the gentle elution technique. The isolated agents may be definitively identified by live culture. Infectious agents which can be isolated by this technique include slow viruses, malaria and infectious yeast.

In the bacteriophage library technique, a binding ligand (e.g., antibody, receptor) is attached to a solid support such as a microtiter plate. This is often accomplished by biotinylation of the ligand, and subsequent immobilization to the plate via a streptavidin bridge. Thus, by using the modified streptavidin of the present invention, the high affinity phages can be released from the microtiter-plates together with the modified biotinylated ligand, by gentle addition of biotin. The recovered high-affinity peptides bound to the phage can then be enriched by subsequent infection of bacteria, and by established phage library procedures.

Purification procedures in which the method may be used include those conventionally used to separate cells, nucleic acids, proteins and other biomaterials, organic compounds, etc.. The method of the invention may also be used to remove immobilized enzymes thus creating a reversible enzyme reactor; and to immobilize cells to column material thus creating cell-based reactor systems. The method may also be used for isolation followed by elution of antigen/antibody-complexes for further downstream analysis like Mass-spectroscopy. Applications in high throughput screening are also applicable.

Another embodiment of the invention is directed to methods involving nucleic acids. Such methods include purification, DNA-based assays, sequencing, in vitro amplifications, etc.. Through the use of the modified biotin/modified streptavidin complex the nucleic acids can be reversibly immobilized to a solid phase. This is contrary to the conventional methods involving biotin/streptavidin binding where the biotinylated strand will stay immobilized on the solid phase. A preferred use of the method is in a procedure for the regeneration of probes for DNA arrays. The immobilized nucleic acid may be single or double stranded and it may comprised cloned sequence or random sequence. Biotinylated nucleic acids are readily prepared using procedures known in the art.

All parameters involved in the attachment and release system described herein may vary dependent on targets/cell type to be isolated, the ligand system or antigen/antibody used, the modified biotin and streptavidin used, and also the type of solid phase used e.g. size of the magnetic beads. All conditions used may readily be determined by those skilled in the art for any given target and binding pairs used.

Conditions for the displacement may be varied as appropriate. The step of "reacting" the linkage with the displacement ligand e.g. free biotin (or fragment thereof) may take place in any convenient or desired way. The "reaction mixture" or the sample containing the linkage, conveniently in an aqueous medium, may simply be contacted with the displacement ligand, e. g. the displacement ligand may simply be added to a sample, and the reaction mixture allowed to stand under appropriate conditions for a time interval to allow the displacement ligand to bind, and one or both of the components of the linkage may then be separated.

The amount of displacement ligand required for optimal cleavage will of course vary depending upon the entities bound, their ratio, and the number or quantity of entities e. g. cells requiring isolation, and can readily be determined according to need.

For example, in the case of positive cell isolation mentioned above, the ratio of magnetic particles to target cells may vary in different systems and with different applications, and different amounts of the displacement ligand will accordingly be required to detach the cell from the particles. An example concentration could be 1 mM, 5 mM, or 10 mM. Concentration of free biotin, in the range of from about 0.1 to 10 mM, preferably 2 to 5 mM, has been found to be effective

Conditions for detachment may also be varied as appropriate. Typically, incubation with free biotin will be effective at temperatures in the range from about 0 °C (on ice) to 37 °C, preferably at room temperature for non-phagocytic cells. Phagocytic cells must be incubated from 0 °C (on ice) to 2-8 °C (cold room).

Incubation times will vary depending on the temperature, materials, and concentrations used and may readily be determined by those skilled in the art for any given set of conditions. Short incubation times are attractive for the user. Typically, incubation times will range from about 2 to about 30 minutes, preferably from about 5 to 10 minutes.

Thus rosetted cells, suspended in a suitable medium, may simply be incubated with 2 to 5 mM free biotin at ambient temperature for 5 to 10 minutes for the cells with the biotinylated ligand still attached to be released from the biotin-binding solid support.

In some cases, it may be desirable to assist reversal of the linkage for example by gentle stirring or mixing e. g. pipetting in order to assist breakage of a linkage destabilized by the displacement ligand binding. Best results are obtained by incubating on an apparatus providing both gentle tilting and rotation.

The advantages of this more general method of reversing antibody-target ligand linkage are self-evident and include the advantages of being more convenient, less time consuming and laborious to develop and therefore more cost-effective. Also the method of the present invention is carried out under very mild/gentle conditions which neither lead to the destruction or loss of activity of either the antibody or target ligand components involved in the linkage nor affect the life or native status of any cells involved. Another advantage of the method of the present invention is that the biotinylated ligands left on the cell surface can immediately be involved in downstream applications.

KITS

An alternative embodiment of the invention is directed towards kits. The kits can comprise a solid support, a ligand, and a displacement reagent. The solid support can generally be any solid support. For example, the solid support can be particulate, or magnetic particles. The solid support can further comprise at least one modified streptavidin such as nitro-streptavidin. The ligand can be directed against a specific target carrying a modified biotin, preferably desthiobiotin, or even more preferably DSB-X-biotin. The ligand can bind to the modified streptavidin on the solid support. The displacement reagent can generally be any material sufficient to displace the ligand from the solid support. For example, the displacement reagent can be biotin.

The kit can alternatively comprise reagents for labeling ligands with a modified biotin.

The following examples are included to demonstrate preferred embodiments of the invention. It should be appreciated by those of skill in the art that the techniques disclosed in the examples which follow represent techniques discovered by the inventor(s) to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the scope of the invention.

### EXAMPLES

ABBREVIATIONS

The following abbreviations are used in the experiments:

BSA = Bovine Serum Albumin

DSB = Desthiobiotin

DMSO= Dimethylsulfoxide

DPBS = Dulbecco's PBS

Dynabeads-NSA = Nitro-streptavidin coated Dynabeads

Dynabeads-SA = Streptavidin coated Dynabeads

FCS = Fetal Calf Serum

EDC/NHS = 1-Ethyl-3-[3-dimethylaminopropyl]carbodiimide hydrochloride/N-hydroxysuccinimide

PBS = Phosphate Buffered Saline

RT = Room temperature

RU = Relative Units (RU)

Example 1: BiaCore data of Desthiobiotin-X/Streptavidin (DSB-X/SA)

### interactions.

MATERIALS AND METHODS

1. Immobilization procedure:

CM5 chips (BiaCore®, BiaCore AB, Uppsala, Sweden) were activated for binding of ligands such as streptavidin or CaptAvidin by using an Amine Coupling Kit from BiaCore. The procedure was followed as provided by the manufacturer. Shortly, EDC/NHS were exposed to the CM5 chip with a flow of 5 µl/minute for 7 minutes. The ligands were then exposed to the activated CM5 surface with a flow of 5 µl/minute for 7 minutes. The excessive reactive groups were deactivated by ethanolamine with a flow of 5 µl/minute for 7 minutes. The RU of ligands were in the order between 4000 ― 19000 RU.

2. Binding of analytes:

The CM5 chip with covalently bound ligand (either recombinant core streptavidin or CaptAvidin (Nitroavidin)) were exposed to analytes (either biotinylated or DSB-X™ biotinylated antibodies) with a flow of 5 µl/minute at a concentration of 50 ― 400 µg/ml until satisfied binding of analytes. The RU of analytes were in the order between 1000 ― 6000 RU.

3. Release of analytes:

The CM5 chip with covalently bound ligand (streptavidin or CaptAvidin) and affinity bound analytes (either biotinylated or DSB-X biotinylated antibodies) were exposed to free D-Biotin with a flow of 5 µl/minute at a concentration of 50µM ― 10mM.

4. Analysis:

All monitored data were treated in the Bioevaluation program to visualize the interaction of free D-Biotin with the different constellations streptavidin ― DSB-X™ biotinylated antibody, CaptAvidin® ― DSB-X™ biotinylated antibody and CaptAvidin® ― biotinylated antibody.

RESULTS

TABLE 1 shows a comparison of the observed binding properties of DSB-X™ biotinylated antibodies (DSB-X™ Ab) and biotinylated antibodies towards CaptAvidin® and Streptavidin. The time of dissociation is measured as the release of half of the bound molecules by the addition of 10mM free biotin.

| TABLE 1 | | |
|---|---|---|
| Ligand | Immobilized binding-partner | Halftime dissociation |
| DSB-X™ labeled-Ab | CaptAvidin® | < 30 seconds |
| Biotinylated-Ab | CaptAvidin® | > 15 minutes |
| DSBX™ labeled-Ab | Streptavidin | > 8 minutes |
| Biotinylated-Ab | Streptavidin | 2.5 years* |

| | | |
|---|---|---|
| * according to the literature | | |

Example 2: Procedure for Nitration of Streptavidin and coupling to Dynabeads.

MATERIALS AND METHODS

1. Preparation of nitro-streptavidin

Streptavidin (50 mg in 5 ml of 50 mM Tris buffer, pH 8,5) was treated with 60 mM tetranitromethane for 2 hours at 25 °C. Nitro-streptavidin was purified by a NAP™-25 column (GE Healthcare Life Sciences).

2, Preparation of nitro-streptavidin beads

100 mg Dynabeads® M-280 Tosylactivated was washed with 0,1 M phosphate buffer, pH 7,4 (3 X 3 ml) and resuspended in 1,8 ml 0,1 M phosphate buffer, pH 7,4. 200 µl nitro-streptavidin (10 mg/ml in PBS) followed by 1 ml 3 M (NH₄)₂SO₄ in 0,1 M phosphate buffer, pH 7,4 were added to the beads. After 16 hours at 37°C on a roller, the beads were washed with 3 ml 50 mM citrate/phosphate-buffer pH 4,0 and resuspended in 3 ml. 100 µl biotin (10 mg/ml in DMSO) were added to block the unmodified biotin binding sites. After 30 minutes, the beads were washed with 3 ml 50 mM carbonate buffer pH 10 to release any biotin bound to the modified binding sites. The beads were stored in a PBS, 0,1% BSA buffer.

**GENERAL PROCEDURE FOR HANDLING DYNABEADS® AND/OR CELLS**

1, Washing of Dynabeads:

1. Transfer desired amount of beads to an appropriate tube

2. Fill the tube ¾ with wash-buffer, mix to homogeneity (e.g. whirl-mix/vortex)

3. Place the tube in a magnet

4. Remove supernatant

5. Resuspend in wash-buffer to the original volume (10 mg beads/ml)

2. Cell pulling and release

1. Add 1 ml of cells at 10⁷ cells/ml to a appropriate tube

2. Add 0.75 mg of Dynabeads

3. Mix to homogeneity (e.g. whirl-mix/vortex)

4. Incubate for 15 minutes at 2-8 °C on a device with both tilting and rotation

5. Mix gently

6. Place the tube in a magnet (Dynal MPC)

7. Collect the supernatant (combine from each wash cycle)

8. Remove the tube from the magnet

9. Add 1 ml wash-buffer and mix to homogeneity (e.g. whirl-mix/vortex)

10. Repeat step 6 ― 9 once

11. Repeat step 6 - 8 once

12. Add 0.5 ml release-buffer (5 mM d-biotin in PBS)

13. Mix to homogeneity (e.g. whirl-mix/vortex)

14. Incubate for 10 minutes at room temperature on a device with both tilting and rotation

15. Mix to homogeneity (e.g. whirl-mix/vortex)

16. Place the tube in a magnet

17. Collect the supernatant (combine from each release cycle)

18. Remove the tube from the magnet

19. Add 0.5 ml release-buffer and mix to homogeneity (e.g. pipetting)

20. Repeat step 16 ― 17 once

Example 3: Methods used in different cell isolation procedures as shown in Figure 1.

MATERIALS AND METHODS

Antibodies used were mouse anti-CD45 antibody, clone EO1, either DSB-labeled or not, and human anti-mouse IgG antibodies, clone HAM6, DSB-labeled.

I) Culture and washing of cells:

1. Grow Daudi cells in RPMI 1640, 10 % FCS and 1 % Na-pyruvate at 0.3―0.5x10⁶ cells/ml. Split 24 hours before use.

2. Wash cells in was-buffer (DPBS , 0.1 % BSA and 2 mM EDTA). Centrifuge at 300 x g for 8 minutes at 2-8 °C. Resuspend in wash-buffer at 10⁷ - 10⁸ cells/ml.

II) Sensitize cells with antibody:

1. Add 0.5 µg antibody (anti-CD45 antibody, either DSB-labeled or not) per 10⁶ cells to washed cells at 10⁷ - 10⁸ cells/ml

2. Incubate at 2-8 °C for 10-15 minutes (or on ice for 30-45 minutes)

Wash cells once by adding 10 x excess volume of wash-buffer, centrifuge at 300 x g for 8 minutes at 2-8 °C. Resuspend cells in wash-buffer at 10⁷ - 10⁸ cells/ml.

3. Alternative: cells are sensitized with a "two layer antibody complex" by adding DSB-labeled human anti-mouse IgG and anti-CD45 at the same time in ratio 2:1.

III) Washing of Dynabeads:

This is performed by following the General procedures.

IV) Coupling of Dynabeads with antibody:

1. Add 3 µg DSB-labeled antibody (human anti-mouse IgG antibodies or anti-CD45 antibody) per mg Dynabeads coupled with nitro-Streptavidin to washed beads at 10 mg beads/ml.

2. Incubate 10 ― 20 minutes at RT.

3. Fill the tube ¾ with wash-buffer, mix to homogeneity (e.g. whirlmix/vortex)

4. Place the tube in a magnet

5. Remove supernatant

6. Repeat step 3 ― 5 twice.

7. Resuspend in wash-buffer to the original volume

Alternative: beads are coupled with a "two layer antibody complex" by adding DSB-labeled human anti-mouse IgG antibodies and anti-CD45 antibodies at the same time in ratio 1:2.

V) Cell pulling and release:

This is performed by following the General procedures.

Overall Set-up

Method A: Combine washed cells (I) and Dynabeads-NSA coupled with DSB-anti-CD45 antibodies (IV).

Method B: Combine cells sensitized with DSB-anti-CD45 antibodies (II) and washed Dynabeads-NSA (III).

Method C: Combine washed cells (I) and Dynabeads-NSA coupled with a "two layer antibody complex" (IV) (DSB-labeled human anti-mouse IgG antibodies and anti-CD45 antibodies).

Method D: Combine cells sensitized with a "two layer antibody complex" (II)(DSB-labeled human anti-mouse IgG and anti CD45) and washed Dynabeads-NSA (III).

Method E: Combine cells sensitized with anti-CD45-antibodies and Dynabeads coupled with DSB-human anti-mouse IgG antibodies

% isolated cells is calculated by the equation:

100% * (# cells added for pulling - # cells in combined wash solution)/ # cells added for pulling

% released cells is calculated by the equation:

100% *# cells in combined release-solution /(# cells added for pulling - # cells in combined wash-solution)

% cell yield is calculated by the equation:

100% * # cells in combined release-solution/ # cells added for pulling

Where:

# cells added for pulling is taken from step 1 in the General procedure, part 2, cell pulling and release

# cells in combined wash-solution is taken from step 7 in the General procedure, part 2, cell pulling and release

# cells in combined release-solution is taken from step 17 in the General procedure, part 2, cell pulling and release

RESULTS are shown in TABLE 2

| TABLE 2 | | | |
|---|---|---|---|
| Procedure | % Isolated cells | % Released cells | % Cell yield |
| A | 90 | 80 | 72 |
| B | 99 | 80 | 79 |
| c | 90 | 40 | 36 |
| D | 99 | 64 | 63 |
| E | 99 | 80 | 79 |

Example 4: Procedure for cell stimulation and expansion

MATERIALS AND METHODS

I) Isolation of peripheral blood mononuclear cells (PBMC):

1. Follow instructions by manufacturer of density gradient media.

2. Wash cells in wash-buffer (DPBS 0.1 % BSA and 2 mM EDTA). Centrifuge at 300 x g for 8 minutes at 2-8 °C. Resuspend cells in wash-buffer at 10⁷ - 10⁸ cells/ml.

II) Sensitize cells with antibody:

1. Add 0.5 µg DSB-labeled antibody (anti-CD3 antibody, clone SpvT3b) per 10⁶ target cells to washed PBMCs at 10⁷ - 10⁸ cells/ml

2. Incubate at 2-8 °C for 10-15 minutes (or on ice for 30-45 minutes)

3. Wash cells once by adding 10 x excess volume of wash-buffer, centrifuge at 300 x g for 8 minutes at 2-8 °C. Resuspend cells in wash-buffer at 10⁷ - 10⁸ cells/ml.

III) Washing of Dynabeads:

This is performed by following the General procedures.

IV) Cell pulling and release:

This is performed by following the General procedures.

V) Cell stimulation

1. Add 10 x excess X-Vivo 15 to released cells (step 17 in IV). Centrifuge at 300 x g for 8 minutes at 2-8 °C. Resuspend cells in X-Vivo 15 at 10⁶ cells/ml.

2. Transfer 10⁵ cells (100 µl suspension) to 96-well U-bottomed plate

3. Dilute biotin-anti-CD28 antibody (clone L2/93) in X-Vivo 15 to 0,0013 mg/ml

4. Add diluted antibody to cells in 96-well plate: 0 ― 0.026 µg/well (0 ― 20 µl suspension)

5. Dilute washed (III) Dynabeads M-280 Streptavidin to 4x10⁶ beads/ml in X-Vivo 15 (10 mg/ml = 6.8x10⁸ beads/ml)

6. Add diluted beads to cells in 96-well plate: 2x10⁵ ― 2x10⁶ beads/well (5 ― 50 µl suspension).

7. Add X-Vivo 15 to a total volume of 200 µl/well

8. Incubate at 37 °C in a CO₂-incubator for 48 hours

9. Pulse for 24 hours with 20 µl of ³H-thymidine (5 µCi/ml final concentration).

10. Harvest and count.

RESULTS are shown in FIG. 5. The graph shows proliferation of CD3⁺ T cells after stimulation with Dynabeads® M280 SA and biotin-anti-CD28 after isolation of CD3⁺ T-cells using DSB-linked-anti-CD3 and NSA-Beads. Pulsed after three days of stimulation, harvested after 4 days, as described in Example 4.

Example 5: Procedure for Double Detach

MATERIALS AND METHODS

Dynabeads-NSA were used to pull cells stained with anti-CD8-X-DSB (desthiobiotin-X coupled anti-CD8 mAb, IgM).

20 minutes cell pulling at 2 ― 8 °C, 5x10⁷ beads/ml and 5x10⁶ cells/ml

3x wash

15 minutes release at room temperature with 1 unit DETACHaBEAD®, or 0.5 mM biotin or a combination of both.

Cells were washed once after release to remove DETACHaBEAD®.

Cells were then stained with Fluorescein isothiocyanate labeled anti-CD3 (anti-CD3-FITC) and Phycoerythrin labeled anti-CD8 (anti-CD8-PE) before isolation, after depletion and after release

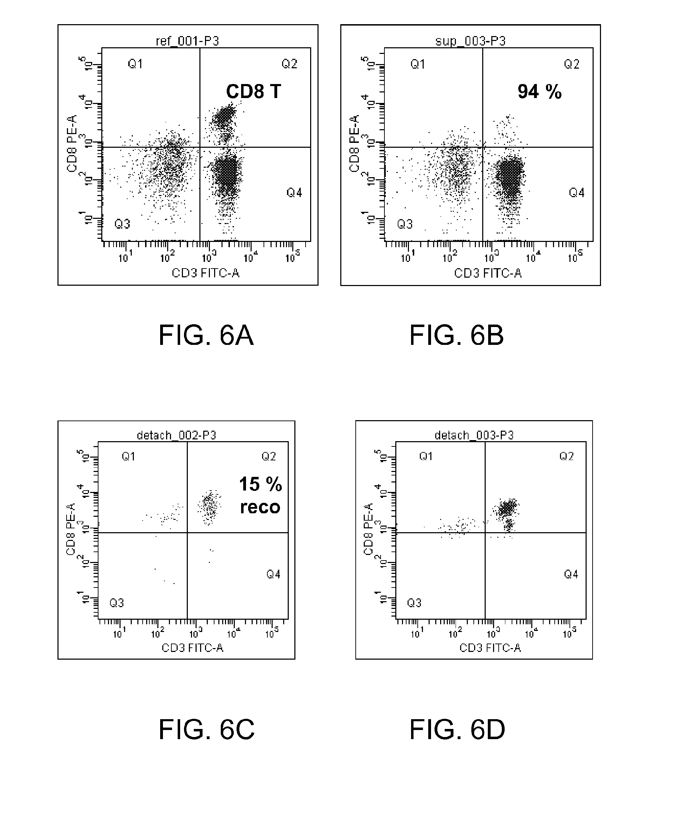

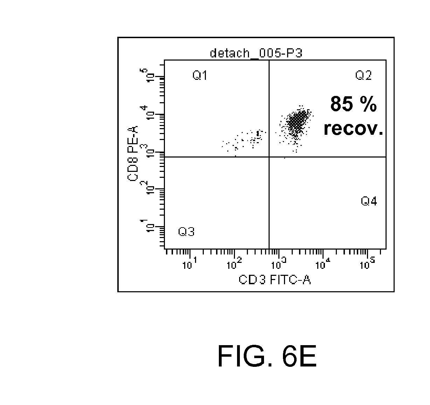

RESULTS are shown in FIG. 6. The graph shows flow staining (anti-CD3-FITC vs. anti-CD8-PE) of human mononuclear cells before isolation (upper left histogram) and after depletion of CD8⁺ cells with Dynabeads-NSA + anti-CD8-X-DSB(upper right histogram). Isolated cells were released from the beads either with DETACHaBEAD® (middle left histogram), d-biotin (middle right histogram) or a combination of DETACHaBEAD® + d-biotin (lower histogram).

DISCUSSION

The release was performed under sub-optimal conditions both for DETACHaBEAD® (optimal 45 minutes at RT) and biotin (optimal above 1 mM), giving sub-optimal release results. Nevertheless, a synergy between two different release mechanisms (on the same binding entity) was observed. This suggests that a "Double detach" approach may be attractive

All of the compositions and/or methods disclosed and claimed herein can be made and executed without undue experimentation in light of the present disclosure. While the compositions and methods of this invention have been described in terms of preferred embodiments, it will be apparent to those of skill in the art that variations may be applied to the compositions and/or methods and in the steps or in the sequence of steps of the methods described herein without departing from the concept and scope of the invention. More specifically, it will be apparent that certain agents which are both chemically and physiologically related may be substituted for the agents described herein while the same or similar results would be achieved. All such similar substitutes and modifications apparent to those skilled in the art are deemed to be within the scope and concept of the invention.

The following items are preferred embodiments of the invention:
1. A method of reversibly immobilizing a modified biotin compound to a support, the method comprising:
   providing a first compound comprising a modified biotin compound;
   providing a solid support comprising a modified biotin-binding compound; and
   contacting the first compound and the solid support to form an immobilized material;
   wherein: the affinity of the modified biotin compound to streptavidin is less than the affinity of biotin to streptavidin; and the affinity of biotin to the modified biotin-binding compound is less than the affinity of biotin to streptavidin.
2. The method of item 1, further comprising contacting the immobilized material with a displacement molecule to release the first compound.
3. The method of item 1, further comprising contacting the immobilized material with a free biotin or a derivative thereof to release the first compound.
4. The method of item 1, wherein the modified biotin-binding compound comprises nitro-streptavidin or derivatives thereof.
5. The method of item 1, wherein the modified biotin compound comprises desthiobiotin, DSB-X biotin, or a derivative thereof.
6. The method of item 1, wherein the solid support is selected from the group consisting of surfaces of plastic, glass, ceramics, silicone, metal, cellulose, and gels.
7. The method of item 1, wherein the solid support is a particle or a magnetic particle.
8. The method of item 1, wherein the modified biotin compound is bound or linked to a protein, peptide, nucleic acid, oligosaccharide, glycoprotein, lipid, carbohydrate, hormone, toxin, derivatives thereof, or combinations thereof.
9. A method of reversibly immobilizing a modified biotin-binding compound to a support, the method comprising:
   providing a first compound comprising a modified biotin-binding compound;
   providing a solid support comprising a modified biotin compound; and
   contacting the first compound and the solid support to form an immobilized material;
   wherein: the affinity of the modified biotin compound to streptavidin is less than the affinity of biotin to streptavidin; and the affinity of biotin to the modified biotin-binding compound is less than the affinity of biotin to streptavidin.
10. The method of item 9, further comprising contacting the immobilized material with a displacement molecule to release the first compound.
11. The method of item 9, further comprising contacting the immobilized material with a free biotin or a derivative thereof to release the first compound.
12. The method of item 9, wherein the modified biotin-binding compound comprises nitro-streptavidin or a derivative thereof.
13. The method of item 9, wherein the modified biotin compound comprises desthiobiotin, DSB-X biotin, or a derivative thereof.
14. The method of item 9, wherein the solid support is selected from the group consisting of surfaces of plastic, glass, ceramics, silicone, metal, cellulose, and gels.
15. The method of item 9, wherein the solid support is a particle or a magnetic particle.
16. The method of item 9, wherein the modified biotin compound is bound or linked to a protein, peptide, nucleic acid, oligosaccharide, glycoprotein, lipid, carbohydrate, hormone, toxin, derivatives thereof, or combinations thereof.
17. A kit comprising:
   (1) a biotin-binding compound attached to a solid support;
   (2) a modified biotin compound bound or linked to at least one biological entity or a reagent for biotinylation of a biological entity with a modified biotin; and
   (3) and free biotin or derivatives thereof,
      wherein the affinity of the modified biotin compound to streptavidin is less than the affinity of biotin to streptavidin, and the affinity of biotin to the modified biotin-binding compound is less than the affinity of biotin to streptavidin
18. The kit of item 17, wherein the biotin-binding compound is nitro-streptavidin.
19. The kit of item 17, wherein the modified biotin comprises desthiobiotin, DSB-X biotin, or a derivative thereof.
20. The kit of item 17, wherein the solid support comprises magnetic particles.
21. A kit comprising:
   (1) A modified biotin compound attached to a solid support;
   (2) a modified biotin-binding compound bound or linked to at least one biological entity or a reagent for biotinylation of a biological entity with a modified biotin;
   (3) and free biotin or derivatives thereof,
      wherein the affinity of the modified biotin compound to streptavidin is less than the affinity of biotin to streptavidin, and the affinity of biotin to the modified biotin-binding compound is less than the affinity of biotin to streptavidin
22. The kit of item 21, wherein the biotin-binding compound is nitro-streptavidin.
23. The kit of item 21, wherein the modified biotin comprises desthiobiotin, DSB-X biotin, or a derivative thereof.
24. The kit of item 21, wherein the solid support comprises magnetic particles.

## Claims

1. A method for positively isolating a target cell or sub-cellular compound from a mixed population comprising:
providing a first compound comprising a modified biotin compound;
providing a solid support comprising a modified biotin- binding compound; and
contacting the first compound and the solid support to form an immobilized material;
wherein the modified biotin-binding compound comprises nitro-streptavidin or
derivatives thereof, and
wherein said modified biotin compound comprises desthiobiotin or a derivative thereof like DSB-X-biotin,
or
providing a first compound comprising a modified biotin-binding compound;
providing a solid support comprising a modified biotin compound; and
contacting the first compound and the solid support to form an immobilized material;
wherein the modified biotin-binding compound comprises nitro-streptavidin or
derivatives thereof, and
wherein said modified biotin compound comprises desthiobiotin or a derivative thereof like DSB-X-biotin,
and
wherein the immobilized material is bound or linked to one or more biological entities.

2. The method of claim 1, further comprising contacting the immobilized material with a displacement molecule to release the first compound, preferably wherein said displacement molecule is free biotin or derivatives or fragments thereof.

3. The method of claims 1 or 2 wherein said solid support is selected from the group consisting of surfaces of plastic, glass, ceramics, silicone, metal, cellulose, and gels.

4. The method of claim 3, wherein the solid support is selected from the group consisting of beads, tubes, chips, resins, plates, wells, films, sticks or particles, preferably wherein the solid support comprises magnetic particles.

5. The method of any one of claims 1 to 4, wherein said biological entity is selected from the group comprising a protein, peptide, nucleic acid, oligosaccharide, glycoprotein, lipid, carbohydrate, hormone or toxin or derivatives thereof.

6. The method of claim 5, wherein said biological entity is an antibody or a derivative thereof directed against a target.

7. The method of claim 6, wherein said target is a cell associated molecule or a cell surface molecule present on or within a cell or a group of cells within a mixed cell population or wherein said target is an antibody or a derivative thereof directed against a second target, preferably wherein said second target is a cell associated molecule or a cell surface molecule present on or within a cell or a group of cells within a mixed cell population.

8. The method of claim 6, wherein said target is a molecule associated with a subcellular component within a mixed population.

9. The method of any one of claims 1 to 8, wherein the cell or sub-cellular component is selected from the group comprising a prokaryotic cell, a eukaryotic cell or an organelle or a virus.

10. Use of the method as defined in any one of claims 1 to 9 in a technique of detection, identification, determination, separation and/or isolation or purification of cells from a mixture of components, in particular in the analysis or diagnosis of a sample.

11. The method of any one of claims 1 to 9 for positively isolating a target cell type from a mixed population of cells,
wherein an antibody biotinylated with the modified biotin is bound to an antigen on the target cell,
said biotinylated antibody being attached to the modified biotin-binding compound on a solid support before or after binding to the target cells under conditions suitable for binding thereto,
whereby the solid support and attached cells are separated from the other cells present, and the target cell is released from the solid phase by the addition of a low concentration of free biotin or derivatives or fragments thereof.

12. Use of the target cell carrying modified biotinylated antibodies recovered by method of any one of claims 1 to 11 in further downstream applications, in particular the isolation, stimulation and expansion of target cells.

13. Use of the method of any one of claims 1 to 11 for the delivery of proteins into cells for in vitro studies of either phagocytosis, delivery of toxic or metabolic substances, or delivery of proteins/peptides for degradation and presentation by the MHC 11/HLA complex on antigen presenting cells (APCs).

14. A kit comprising:
(i) nitro-streptavidin attached to a solid support;
(ii) desthiobiotin or a derivative thereof like DSB-X-biotin bound or linked to at least one biological entity or a reagent for biotinylation of a biological entity with desthiobiotin or a derivative thereof like DSB-X-biotin;
and/or
(iv) desthiobiotin or a derivative thereof like DSB-X-biotin attached to a solid support;
(v) nitro-streptavidin bound or linked to at least one biological entity or a reagent for coupling the biological entity with nitro-streptavidin;
(vi) and free biotin or derivatives or fragments thereof.

15. The kit of claim 14, wherein said solid support comprises magnetic particles.
